# EUROPEAN PATENT APPLICATION

(11) **EP 4 527 614 A1**
(43) Date of publication of application: **26.03.2025**
(21) Application number: 23807579.0
(22) Date of filing: 12.05.2023
(51) Int. Cl.: B32B 3/06, A41D 13/00, A42B 3/20, A44B 17/00, A44B 99/00

(54) **FILM LAMINATE, METHOD FOR PRODUCING FILM LAMINATE, PROTECTOR AND METHOD FOR PRODUCING PROTECTOR**

(30) Priority: 19.05.2022 JP 2022082379
(71) Applicant: Dexerials Corporation, Shimotsuke-shi, Tochigi 323-0194 (JP)
(72) Inventor: OISHI, Kiyokazu, Shimotsuke-shi, Tochigi 323-0194 (JP); KATO, Takuya, Shimotsuke-shi, Tochigi 323-0194 (JP)
(74) Representative: Müller-Boré & Partner Patentanwälte PartG mbB
(86) International application number: PCT/JP2023/017966
(87) International publication number: WO 2023/223977

(57) **Abstract**

A film laminate that can achieve sufficient sterilization effects and does not have the risk of damaging the film surface structure is provided. A film laminate 1 includes a plurality of film-shaped members 2 laminated in a separable manner, and columnar protruding members 5 protruding in the direction of lamination of the film-shaped members 2 and holding the film-shaped members 2 at a predetermined interval, wherein the protruding members 5 have engagement portions on the outer circumference of the protruding members 5 to which the film-shaped members 2 are engaged at the predetermined interval, the film-shaped members 2 have insertion holes 4 through which the protruding members 5 are inserted and removed as desired, and the film-shaped members 2 are held at the predetermined interval and laminated by the protruding members 5 being inserted through the insertion holes 4 and the film-shaped members 2 being engaged with the engagement portions.

## Description

### TECHNICAL FIELD

The present technology relates to a film laminate including multiple film-shaped members laminated at a predetermined interval, a method for manufacturing a film laminate, protective equipment using a film laminate, and method for manufacturing protective equipment. This application claims priority based on Japanese Patent Application No. 2022-082379 filed in Japan on May 19, 2022, which is incorporated herein by reference.

### BACKGROUND ART

Personal protective equipment is widely used as a means of preventing occupational infections among medical personnel. In particular, protective equipment such as face shields and protective clothing, which are used to prevent exposure to blood and body fluids, are used during examinations and surgeries and are useful for preventing occupational infections from blood-borne pathogens (HIV, HBV, HCV) and coronaviruses (such as SARS-CoV-2).

However, the surface of this type of protective equipment may become contaminated with blood or body fluids that have splashed off the patient during an examination or surgery. In such an environment where contaminants are flying around, it is necessary to quickly restore visibility while avoiding contact with the contaminants.

In addition, helmet shields used in motorcycle and auto racing, as well as protective eyewear used in painting, have the same problem of frequently dirtying the surface and obstructing visibility.

To solve this problem, it has been conventionally done to laminate several layers of easily separable protective films on the surface of the helmet shield or protective glasses for painting, and when the vision is obstructed by dirt, the topmost protective film is peeled off along with the dirt to restore the vision. With regard to this kind of technology, for example, in Patent Document 1, a protective device for the shield of a helmet is disclosed, which is covered with multiple sheet-shaped protective covers, i.e., disposable visors.

However, if the protective equipment is made as described above, with a simple laminated structure of protective film, reflection occurs at the interface between each of the laminated layers. There are also problems such as a decrease in transmissivity as the number of layers increases.

On the other hand, there is also proposed protective equipment using optical elements with a moth-eye structure with a pitch smaller than the wavelength of visible light and films with low-reflection characteristics to provide excellent visibility (Patent Document 2).

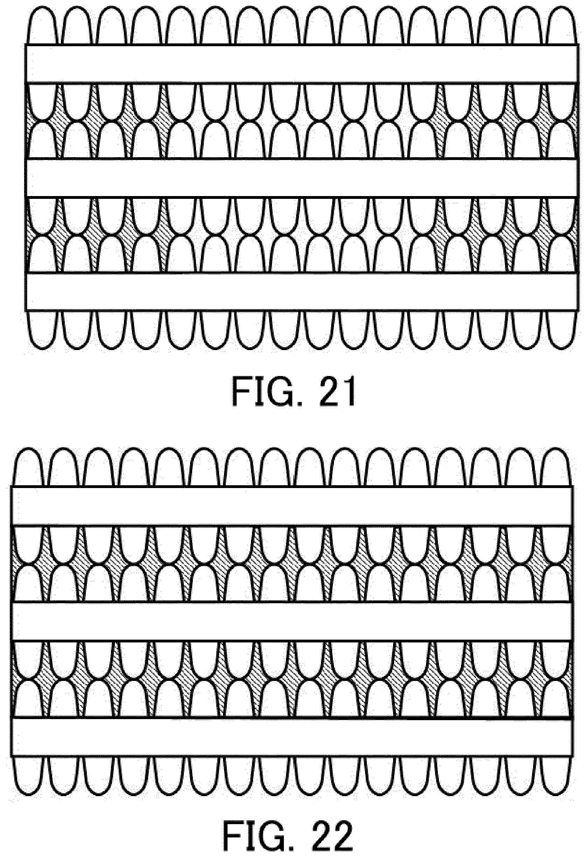

As shown in FIGS. 21 and 22, the laminate described in Patent Document 2 includes multiple film-shaped members having a moth-eye structure consisting of unevenness with a pitch smaller than the visible light wavelength on at least one surface of the base body and is formed by laminating the film-shaped members in a separable manner by fixing them via a pressure-sensitive adhesive layer at least at the edges of the film-shaped members. In the laminate shown in FIG. 21, the pressure-sensitive adhesive layer is provided only at the edges of the film-shaped members. In addition, the laminate shown in FIG. 22 has a pressure-sensitive adhesive layer covering the entire surface of the film-shaped member.

By laminating film-shaped members having the moth-eye structure, it is possible to prevent increases in reflectivity and decreases in transmittance, thereby ensuring visibility. In addition, when the surface of the laminate becomes contaminated with blood or body fluids, or when dirt obscures the vision, the topmost film-shaped member can be peeled off along with the dirt to restore visibility.

### CITATION LIST

### PATENT LITERATURE

Patent document 1: JP 2000-192322 A
Patent document 2: JP 2015-057317 A

### SUMMARY OF INVENTION

### TECHNICAL PROBLEM

When protective equipment provided with such laminated film-shaped members is used for medical purposes, the surfaces of each film-shaped member are sterilized by gas sterilization or other methods. Then, when the contaminated topmost film-shaped member is peeled off, a new sterile surface is exposed on the outermost surface.

However, in the case of protective equipment that uses a laminate in which the film-shaped members are laminated and fixed together with a pressure-sensitive adhesive layer, when sterilization is carried out using gas or other means, the surfaces of the film-shaped members are not exposed in the areas bonded with the pressure-sensitive adhesive layer, so the sterilizing gas cannot flow in and the unexposed surface is not sterilized.

In the configuration shown in FIG. 21, the central part of the film without the pressure-sensitive adhesive layer can be sterilized, but the outer edge of the film with the pressure-sensitive adhesive layer cannot be sterilized. Furthermore, in the configuration shown in FIG. 21, if the pressure-sensitive adhesive layer covers the entire circumference of the film, the front surface and the back surface of the film enclosed by the pressure-sensitive adhesive layer will not be sterilized. In the configuration shown in FIG. 22, only the topmost surface and the bottom surface of the laminate are sterilized, and the front surface and the back surface of the film with the pressure-sensitive adhesive layer in between cannot be sterilized.

Therefore, when the contaminated topmost film-shaped member is peeled off, a film-shaped member that has not been sterilized in some areas or has not been sterilized at all is exposed; which means that the sterilization process was not fully effective.

In the case of a transparent laminate with a moth-eye structure formed on the film surface laminated using a pressure-sensitive adhesive, there is a risk that the moth-eye structure may be destroyed when peeling off the individual film-shaped member, depending on the adhesive strength.

Therefore, an object of the present technology is to provide a film laminate capable of achieving sufficient sterilization effects without the risk of damaging the film surface structure, as well as a method for manufacturing a film laminate, protective equipment, and a method for manufacturing protective equipment.

### SOLUTION TO PROBLEM

In order to solve the problem, a film laminate according to the present technology includes a plurality of film-shaped members laminated in a separable manner, and columnar protruding members protruding in the direction of lamination of the film-shaped members and holding the film-shaped members at a predetermined interval, wherein the protruding members have engagement portions on the outer circumference of the protruding members to which the film-shaped members are engaged at the predetermined interval, the film-shaped members have insertion holes through which the protruding members are inserted and removed as desired, and the film-shaped members are held at the predetermined interval and laminated by the protruding members being inserted through the insertion holes and the film-shaped members being engaged with the engagement portions.

A method for manufacturing a film laminate according to the present technology includes a step of forming film-shaped members having insertion holes, a step of forming protruding members having engagement portions that engage with the film-shaped members, and a step of inserting the protruding members through the insertion holes of the film-shaped members, causing the film-shaped members to be engaged with the engagement portions, and laminating the film-shaped members at a predetermined interval.

In addition, protective equipment according to the present technology is protective equipment in which a film laminate is attached to a part corresponding to the face or eyes of a user, wherein the film laminate is as described above.

In addition, a method for manufacturing protective equipment according to the present technology is a method for manufacturing protective equipment in which a film laminate is attached to a part corresponding to the face or eyes of a user, including a step of forming a film laminate, and a step of attaching the film laminate to protective equipment, wherein the film laminate is as described above.

### ADVANTAGEOUS EFFECTS OF INVENTION

According to the present technology, the film laminate can expose the entire front and back surfaces of each film-shaped member to sterilizing gas, and when a film-shaped member is peeled off, it is always possible to expose a film-shaped member that has been sterilized on all surfaces.

### BRIEF DESCRIPTION OF DRAWINGS

FIG. 1 is a disassembled perspective view of a film laminate according to the present technology.
FIG. 2 is side views illustrating the protruding members, in which FIG. 2A shows cylindrical protrusions, FIG. 2B shows ring-shaped protrusions, FIG. 2C shows spherical protrusions, FIG. 2D shows protrusions that are gradually enlarged from the tip to the base, and FIG. 2E shows protrusions with a groove cut into it in a circumferential direction.
FIG. 3 is a side view showing an example of the dimensions of a protruding member with cylindrical protrusions.
FIG. 4 is a side view of a protruding member with ring-shaped protrusions and film-shaped members engaged with the protrusions.
FIG. 5 shows an example of a configuration with four insertion holes arranged at equal intervals along the top edge of a film-shaped member with a roughly rectangular shape.
FIG. 6 is a perspective view illustrating the slits formed around the insertion holes.
FIG. 7 is a cross-sectional view of a film-shaped member constituting a film laminate according to the present technology.
FIG. 8 is a cross-sectional view of another film-shaped member constituting a film laminate according to the present technology.
FIG. 9 is a perspective view of another film-shaped member constituting a film laminate according to the present technology.
FIG. 10 is a perspective view of a roll master for transfer printing, on which a pattern of anti-reflection layer composed of a moth-eye structure has been formed.

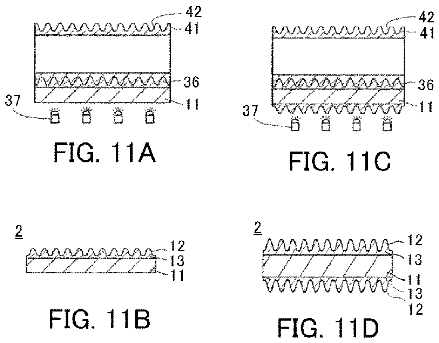

FIG. 11 shows cross-sectional views illustrating the steps of forming an anti-reflection layer composed of a moth-eye structure, in which FIG. 11A shows a state wherein a side of the base material to which the transfer material has been applied is brought into close contact with the roll master, FIG. 11B shows a film-shaped member on which the moth-eye structure has been transferred to one side, FIG. 11C shows a state wherein the transfer material is applied to the other side of the film-shaped member other than the side to which the moth-eye structure has been transferred, and the other side is brought into close contact with the roll master, and FIG. 11D shows the film-shaped member with the moth-eye structure transferred to both sides.
FIG. 12 is an exterior perspective view illustrating a protective suit for medical use as an example of protective equipment.
FIG. 13 is a cross-sectional view illustrating one configuration example of a film laminate.
FIG. 14 is a cross-sectional view illustrating another example of a film laminate.
FIG. 15 is a cross-sectional view illustrating an example of a step of attaching a film laminate to protective equipment.
FIG. 16 is a cross-sectional view illustrating another example of a step of attaching a film laminate to protective equipment.
FIG. 17 is a cross-sectional view illustrating another example of a step of attaching a film laminate to protective equipment.
FIG. 18 is an explanatory view of the composition of a film laminate sample made by laminating film-shaped members using hook members.
FIG. 19 is an explanatory view of the composition of a film laminate sample made by laminating film-shaped members using pressure-sensitive adhesive applied to the full surface, in which FIG. 19A is a plan view, and FIG. 19B is a cross-sectional view.
FIG. 20 shows a film laminate sample for which pressure-sensitive adhesive is applied to one side edge of the film-shaped member for lamination, in which FIG. 20A is a plan view and FIG. 20B is a cross-sectional view.
FIG. 21 is a cross-sectional view of a laminate in which multiple film-shaped members are laminated using pressure-sensitive adhesive layers provided on the edges of the film-shaped member. FIG. 22 is a cross-sectional view of a laminate in which multiple film-shaped members are laminated together using pressure-sensitive adhesive layers provided on the entire surfaces of the film-shaped members.

### DESCRIPTION OF EMBODIMENTS

Embodiments of a film laminate, a manufacturing method for a film laminate, protective equipment, and a manufacturing method for protective equipment according to the present technology will now be more particularly described with reference to the accompanying drawings. It should be noted that the present technology is not limited to the embodiments described below and various modifications can be added to the embodiment without departing from the scope of the present technology. The features shown in the drawings are illustrated schematically and are not intended to be drawn to scale. Actual dimensions should be determined in consideration of the following description. Moreover, those skilled in the art will appreciate that dimensional relations and proportions may be different among the drawings in some parts.

### FILM LAMINATE

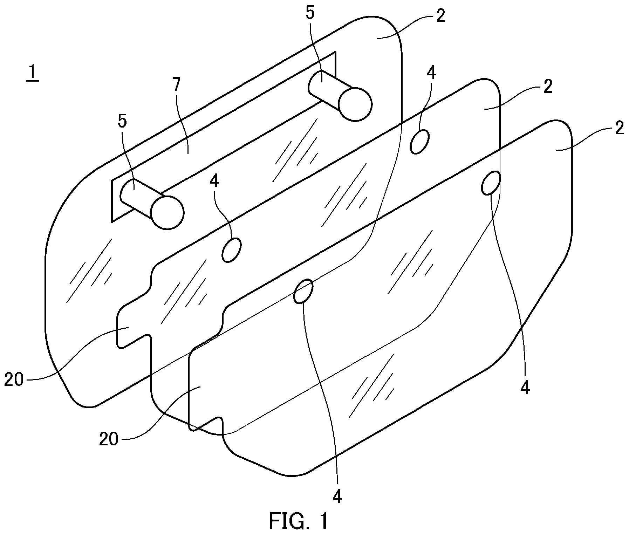

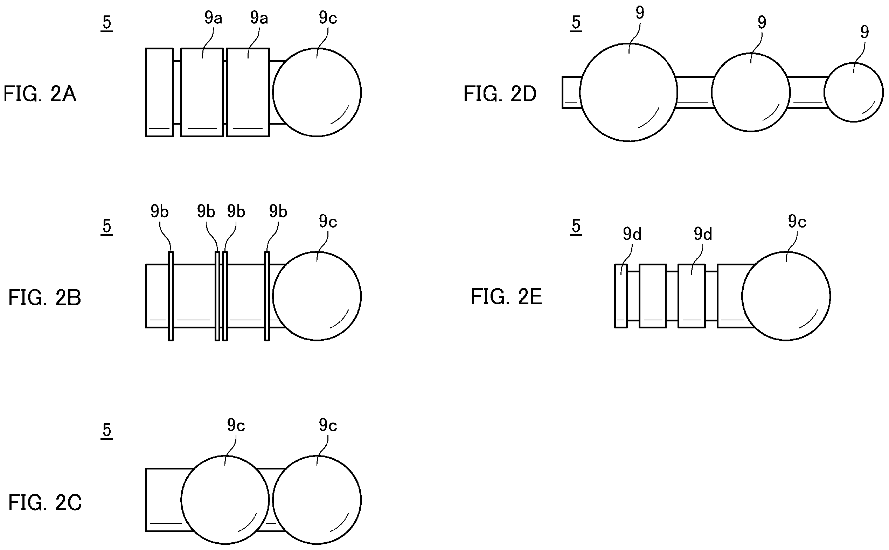

As shown in FIG. 1, a film laminate 1 according to the present technology includes a plurality of film-shaped members 2 laminated in a separable manner, and columnar protruding members 5 protruding in the direction of lamination of the film-shaped members 2 and holding the film-shaped members 2 at a predetermined interval. As shown in FIG. 2, the outer circumference of the protruding members 5 is provided with engagement portions to which the film-shaped members 2 are engaged at the predetermined interval. The film-shaped members 2 have insertion holes 4 through which the protruding members 5 can be inserted and removed. When the protruding members 5 is inserted through the insertion holes 4, the film-shaped members 2 are engaged with the engagement portions. As a result, the film-shaped members 2 are laminated and held at a predetermined interval.

The film-shaped member 2 is a flexible transparent sheet. Each of the film-shaped members 2 has the insertion holes 4, and the film-shaped members 2 are laminated by inserting the protruding members 5 through the insertion holes 4. Each film-shaped member 2 is held at a predetermined interval by being engaged with the engagement portions provided on the outer circumference of the protruding members 5. This allows the multiple film-shaped members 2 to be laminated at a predetermined distance from each other, while also exposing the entire front and back surfaces of each film-shaped member 2.

Therefore, the film laminate 1 makes it possible to expose the entire front and back surfaces of each film-shaped member 2 to a sterilizing gas; when the film-shaped member 2 is peeled off, it is always possible to expose a film-shaped member 2 that has been sterilized on all its surfaces.

In medical applications, if the surface becomes contaminated with blood or body fluids splashed from the patient during an examination or surgery, the topmost film-shaped member 2 can be peeled off to quickly restore visibility while avoiding contact with the contaminant. At this time, since the entire surface of the film-shaped member 2 exposed by peeling off the film laminate 1 has been sterilized, the risk of infection to the patient, the examinee, and the medical personnel can be reduced. In addition, since the entire back surface of the peeled-off film-shaped member 2 has also been sterilized, there is no risk of exposing the patient, examinee, and medical personnel to unsterilized areas when the film-shaped member 2 is peeled off or disposed of.

### PROTRUDING MEMBER

The protruding members 5 that support the film-shaped members 2 are column-shaped and, as shown in FIG. 1 for example, are erected near both ends of the rectangular plate-shaped base 7, protruding in the direction of the lamination of the film-shaped members 2. The protruding members 5 can be molded using, e.g., a known engineering plastic. The molding method may be a known molding method such as metal mold molding or cutting. The protruding members 5 can also be formed by molding them integrally with the base 7 or by forming them as separate parts and then connecting them to the base 7 using an adhesive or other method.

### ENGAGEMENT PORTION

The protruding members 5 also have engagement portions that engage with the film-shaped members 2 disposed at predetermined intervals on the outer circumference. The engagement portions are formed as a plurality of protrusions 9 that protrude in the circumferential direction of the protruding members 5, and the insertion holes 4 of the film-shaped member 2 through which the protruding members 5 are inserted are held between the protrusions 9. This restricts the movement of the film-shaped members 2 in the erecting direction of the protruding members 5 and holds them at a predetermined interval.

The shape of the protrusions 9 is not particularly restricted as long as the protrusions 9 can hold the film-shaped member 2 in place and can be cylindrical as shown in FIG. 2A. The engagement portions can hold the film-shaped member 2 by having multiple cylindrical protrusions 9a at a predetermined interval and holding the film-shaped member 2 between the protrusions 9a. The interval between the film-shaped members 2 is defined by the width of the protrusions 9a that extend in the erecting direction of the protruding members 5.

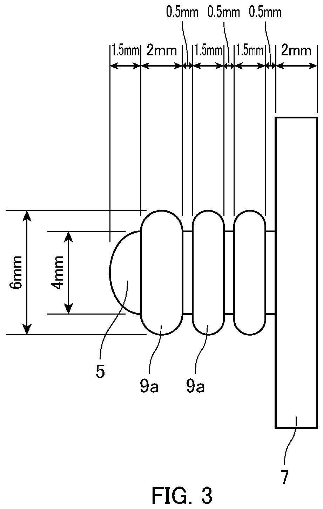

FIG. 3 is a side view showing an example of the dimensions of the protruding members 5 with protrusions 9a. In the protruding members 5 shown in FIG. 3, the film-shaped members 2 with a thickness of 0.5 mm are laminated at intervals of 1.5 mm. As shown in FIG. 3, the protrusions 9a may also be a circular ring shape with a circular arc side. This allows the film-shaped member 2 to be inserted and removed smoothly and also prevents damage during insertion. In addition, the rounded side of the engagement portion enhances safety for the user and other members and also creates a sense of security.

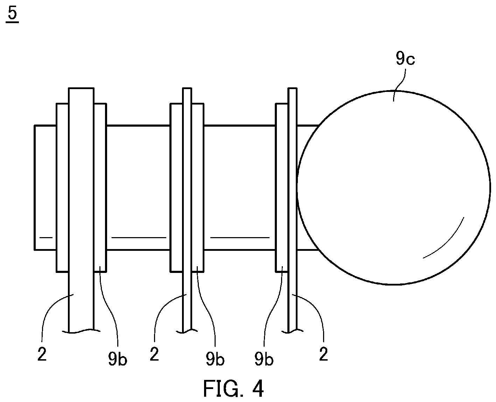

In addition, as shown in FIG. 2B, the shape of the protrusions 9 may be ring-shaped. By providing multiple ring-shaped protrusions 9b, the engagement portion can restrict the movement of the film-shaped member 2 toward the base or tip of the protruding members 5 and restrict the movement of the film-shaped member 2 by holding the film-shaped member 2 between adjacent two protrusions 9b. As shown in the example shown in FIG. 2B, in the case of holding three film-shaped members 2, the protrusion 9b formed on the most base side of the protruding member 5 restricts the movement of the bottommost film-shaped member 2 toward the tip side of the protruding member 5, the protrusion 9b formed on the most tip side of the protruding members 5 restricts the movement of the topmost layer film-shaped member 2 toward the base side of the protruding member 5, and a pair of protrusions 9b formed on the intermediate portion of the protruding members 5 restricts the movement of the intermediate film-shaped member 2 toward the base side and the tip side of the protruding member 5. The interval between the film-shaped members 2 is defined by the interval between the protrusions 9b that extend in the erecting direction of the protruding members 5 (see FIG. 4).

Alternatively, as shown in FIG. 2C, the protrusions 9 may be spherical in shape. The engagement portion can hold the film-shaped members 2 by providing multiple spherical protrusions 9c and engaging the film-shaped members 2 between the protrusions 9c. The interval between the film-shaped members 2 is defined by the diameter of the protrusions 9c and the interval between the protrusions 9c in the erecting direction of the protruding members 5. The spherical protrusions 9c allow the film-shaped members 2 to be inserted and removed smoothly.

There is no particular restriction on the interval between each film-shaped member 2, as long as it is possible to prevent the film-shaped members 2 from being in close contact and to expose the entire front and back surfaces to the sterilizing gas, and, for example, an interval of 0.1 mm or more is preferable. Further, if the interval is too wide, there is a risk of foreign matter being entered when using protective equipment 21, so an interval of 5 mm or less is preferable, and it is even better to make it as narrow as possible. In addition, the interval between each film-shaped member 2 may be constant or different. For example, the interval between the topmost film-shaped member 2 and the second film-shaped member 2 may be different from the interval between the second film-shaped member 2 and the bottommost film-shaped member 2.

In addition, the protruding member 5 may have a spherical protrusion 9c at its tip. By providing a spherical protrusions 9c at the tip, it is possible to smoothly insert and remove the film-shaped member 2 and also to prevent damage during insertion. In addition, the rounded tip of the protruding members 5 enhances safety for the user and other members and also creates a sense of security.

As shown in FIG. 2D, in the protruding members 5 having a plurality of protrusions 9, the diameter of the protrusions 9 may gradually increase from the tip to the base, and the aperture of the insertion hole 4 provided in the film-shaped member 2 may have an aperture diameter corresponding to the diameter of the protrusions 9 at the engagement positions of the film-shaped member 2. This prevents the topmost film-shaped member 2 and the intermediate film-shaped member 2 from moving toward the base side from the predetermined engagement position. In addition, if the laminating order of the film-shaped members 2 is determined in advance, it is possible to prevent the topmost film-shaped member 2 and intermediate film-shaped member 2 from being engaged with a wrong position shifted to the base side from the predetermined engagement position.

Instead of providing the protrusions 9, the engagement portions may also be formed as a plurality of grooves 9d that are recessed around the circumference of the protruding members 5, as shown in FIG. 2E, and the area around the insertion holes 4 of the film-shaped member 2 through which the protruding members 5 are inserted may be engaged with the grooves 9d. The engagement portions may also be composed of a combination of the protrusions 9a to 9c and grooves 9d as appropriate.

### FILM-SHAPED MEMBER

The film-shaped member 2 is a flexible transparent sheet. There is no particular restriction on the shape of the film-shaped member 2, and it can be selected as appropriate according to the protective equipment 21 to be applied, e.g., as a roughly rectangular shape as shown in FIG. 1. Each of the film-shaped members 2 has insertion holes 4 through which the protruding members 5 are inserted and which are also used to engage the protruding member 5 in place.

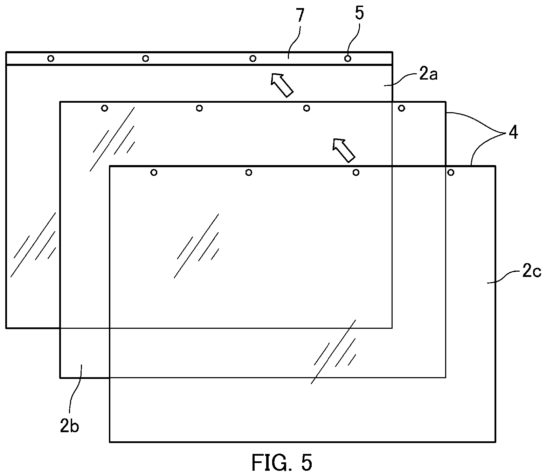

The protruding member 5 and the insertion holes 4 are provided in such a position and number as to be able to stably hold the film-shaped member 2 at a predetermined interval, and at the same time, not interfere with the user's field of vision within the film-shaped member 2 when in use, and also to allow easy peeling operation. From this perspective, it is preferable to provide the insertion holes 4 on the outer edge of the film-shaped member 2; for example, as shown in FIG. 1, it is preferable to form the insertion holes 4 on both ends of the top edge of the film-shaped member 2, which is formed in a rectangular shape, or as shown in FIG. 5, it is preferable to arrange a plurality of (e.g., four) insertion holes 4 at equal intervals along the top edge of the rectangular film-shaped member 2. In addition, the insertion holes 4 may be formed not only on the upper edge of the rectangular film-shaped member 2, but also on the lower edge or side edges, and they may be formed in one or more of these locations or arranged in multiple locations. The protruding members 5 are also formed and arranged according to the forming position of the insertion holes 4. The forming position and number of the protruding members 5 and insertion holes 4 are not limited to those described above and are set as appropriate according to the configuration of the protective equipment 21 and the mounting configuration of the film laminate 1, as well as its intended use, among other factors.

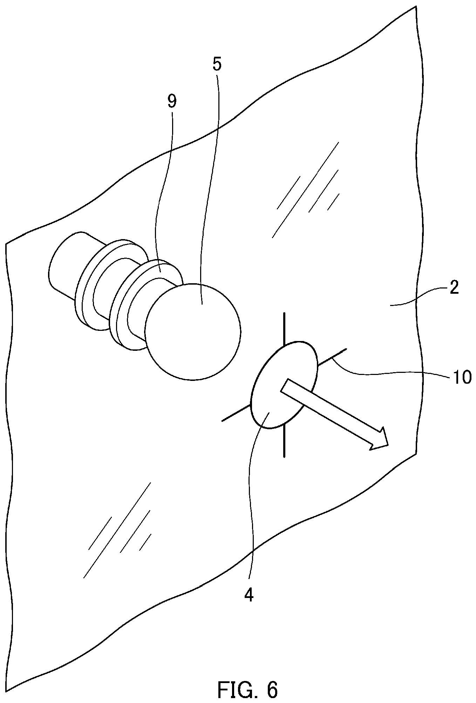

In addition, as shown in FIG. 6, the film-shaped member 2 may have one or more slits 10 formed around the insertion holes 4. As the engagement portion of the protruding members 5 is pressed into the insertion holes 4, the film-shaped member 2 can be inserted and removed more smoothly by forming the slit 10.

The film-shaped member 2 may have a tab 20 for peeling at the outer edge. The tab 20 is a part that is pinched to peel off the film-shaped member 2. It is preferable to give the tab 20 a function to identify the film-shaped member 2 to be peeled off. This makes it possible to prompt the user to peel off the topmost layer film-shaped member 2 and prevents the user from accidentally peeling off the intermediate film-shaped member 2 together with the topmost film-shaped member 2.

One way to identify the topmost film-shaped member 2 is to make the tabs 20 successively smaller from the topmost layer. In other words, as shown in FIG. 1, the tab 20 formed on the topmost film-shaped member 2 is made larger than the tabs 20 on the film-shaped members 2 below it to hide the tabs on the lower film-shaped members 2, so that it is always easy to pinch only the tab 20 on the topmost film-shaped member 2, and it is prevented from accidentally pinching the tabs 20 on the lower film-shaped members 2.

In addition, the position of the tab 20 may be changed for each film-shaped member 2. For example, the topmost film-shaped member 2 has a tab 20 on the right side edge in a front view, and the intermediate film-shaped member 2 has a tab 20 on the left side edge in a front view. In this way, by first pinching the tab 20 on the right side edge, the topmost film-shaped member 2 can be peeled off, and it is possible to prevent the intermediate film-shaped member 2 from being peeled off by mistake.

In addition, it is also possible to identify the film-shaped members 2 by changing the color of the tab 20 for each film-shaped member 2, or by processing physical features such as uneven marks, openings, and notches. In addition, the above identification means may be combined.

Furthermore, the film laminate 1 may have all the same film-shaped members 2 for each layer, or it may have film-shaped members 2 with different functions or optical characteristics. These configurations are selected as appropriate according to the application of the protective equipment 21 to which the film laminate 1 is applied. In addition, the laminating order of the film-shaped members 2 with different functions or characteristics is also set as appropriate according to the application of the protective equipment 21.

### MOTH-EYE STRUCTURE

The film-shaped member 2 is preferably an optical element with an anti-reflection function, in which multiple structures are provided on at least one surface of a flexible transparent base material at a pitch smaller than the wavelength of visible light. The fine uneven structure with an anti-reflection function will be referred to as the "moth-eye structure" in the following. Furthermore, by using a film-shaped member 2 with a moth-eye structure, the film-shaped member 2 can be laminated without impairing visibility.

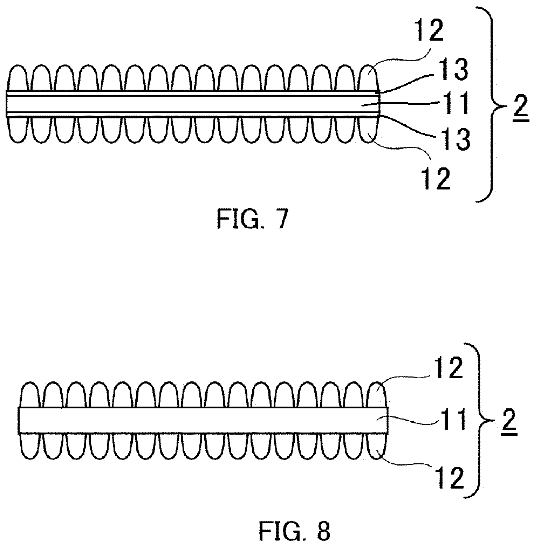

As shown in FIG. 7, the film-shaped member 2 has structures 12 with a pitch smaller than the wavelength of visible light on both sides of a base body 11 via a base layer 13 and thereby has an anti-reflection function on both the front surface and the back surface opposite to each other. The multiple structures 12 are regularly arranged in multiple rows on the front surface and the back surface of the base body 11, on top of the base layer 13. In other words, the front surface and the back surface of the film-shaped member 2 have unevenness made up of the multiple structures 12, i.e., the moth-eye structure. The film-shaped member 2 may have the structures 12 only on the front surface of the base body 11.

By providing such unevenness on the front surface and the back surface of the film-shaped member 2, the protective equipment 21 to which the film laminate 1 is attached will have an excellent optical adjustment function with little wavelength dependence and excellent visibility. In other words, this can contribute to achieving protective equipment 21 with excellent visibility.

Here, "optical adjustment function" refers to adjustment functions for optical characteristics such as transmission characteristics and reflection characteristics. The film-shaped member 2, as an optical element, is transparent to visible light, and the refractive index n thereof is preferably 1.30 to 2.00, and more preferably 1.34 to 2.00. However, the refractive index n is not limited to this range.

In addition, the refractive index of the structures 12 is preferably the same as or approximately the same as the refractive index of the base body 11. This is because the same or approximately the same refractive index will suppress internal reflection and improve contrast.

In FIG. 7, an example is shown in which the structures 12 are formed on the front surface and the back surface of the base body 11 via the base layer 13, and this base layer 13 plays a role in improving the adhesion of the structures 12 to the base body 11. In this case, the base layer 13 is a transparent optical layer integrally molded with the structures 12 on the bottom side of the structures 12 and may be formed by curing an energy ray-curable resin composition, or the like, similar to the structures 12.

In addition, as shown in FIG. 8, the film laminate 1 may use the film-shaped member 2 in which the moth-eye structure of the plurality of structures 12 are directly formed on the base body 11 without the base layer 13.

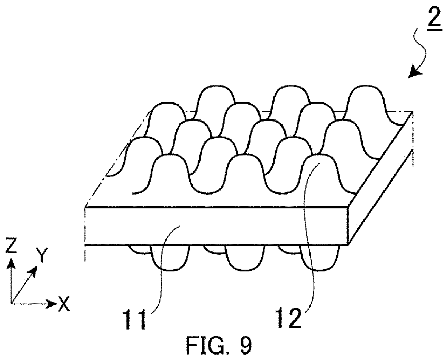

Furthermore, as shown in FIG. 9, the film laminate 1 may use the film-shaped member 2 in which the base body and the structures are integrally molded. The film-shaped member 2 shown in FIG. 9 has the structures 12 integrally molded on both sides of the base body 11.

### BASE BODY

The base body 11 will be further explained here. The base body 11 is composed of, e.g., a transparent base material. For example, the base body 11 is mainly composed of a transparent plastic material but is not limited to these materials.

When using a plastic material as the base body 11, in order to further improve the surface energy, coating properties, slipperiness, and flatness, among other properties, of the plastic material surface, a further undercoating layer not illustrated may be provided by surface treatment. This undercoating layer may include, e.g., organoalkoxymetal compounds, polyesters, acrylic-modified polyesters, and polyurethanes, among others. In addition, in order to obtain the same effect as that of the undercoating layer, it is also possible to carry out corona discharge treatment, UV irradiation treatment, and the like on the surface of the base body 11.

When the base body 11 is a plastic film, the base body 11 can be obtained by, e.g., stretching the above-mentioned resin or dissolving the resin in a solvent and then drying the solution to form a film. The thickness of the base body 11 is preferably selected as appropriate for the application of the film-shaped member 2, and may be, e.g., 10 µm or more and 500 µm or less. The shape of the base body 11 may be, e.g., film-shaped or plate-shaped but is not limited to these shapes. Note that the term "film" includes sheets.

The examples of the material for the base body 11 may include methyl methacrylate (co) polymer, polycarbonate, styrene (co) polymer, methyl methacrylate-styrene copolymer, cellulose diacetate, cellulose triacetate, cellulose acetate butyrate, polyester, polyamide, polyimide, polyethersulfone, polysulfone, polypropylene, polymethylpentene, polyvinyl chloride, polyvinyl acetal, polyetherketone, polyurethane, and glass, but the material is not limited to these.

### STRUCTURE

Next, the structures 12 will be explained. Generally, the wavelength band of visible light is 360 nm to 830 nm, but in this embodiment, the structures 12 are arranged in a regular pattern with a size less than the wavelength band of visible light. From this perspective, the arrangement pitch of the structures 12 shall not exceed 350 nm. The structures 12 may be of various shapes, such as conical, columnar, or needle-shaped.

As described below, the structures 12 are formed by using a roll master exposure device in which a pattern corresponding to the moth-eye structure is formed to transfer the pattern to the transfer material 36, such as an energy ray-curable resin composition coated on the base body 11 and then curing the transfer material 36.

The cured material of the transfer material 36 may have hydrophilic properties. It is preferable that the transfer material 36 contains one or more functional groups having hydrophilic properties. Such functional groups having hydrophilic properties include, e.g., hydroxyl groups, carboxyl groups, and carbonyl groups.

In addition, the energy ray-curable resin product that forms the structures 12 may have different physical properties on both sides of the base body 11. For example, depending on the application, the hydrophobic and hydrophilic properties can be used separately to provide specific surfaces with functions such as anti-fogging.

It is preferable to use a UV ray-curable resin composition as the energy ray-curable resin composition. In addition, the energy ray-curable resin composition may contain fillers or functional additives as needed.

The UV ray-curable resin composition contains, e.g., an acrylate and an initiator.

The UV ray-curable resin composition includes, e.g., monofunctional monomers, difunctional monomers, and polyfunctional monomers, and specifically, it is a mixture of the following materials alone or in combination.

Example of the "monofunctional monomer" may include carboxylic acids (acrylic acid), hydroxy groups (2-hydroxyethyl acrylate, 2-hydroxypropyl acrylate, 4-hydroxybutyl acrylate),alkyl or alicyclic monomers (isobutyl acrylate, t-butyl acrylate, iso-octyl acrylates, lauryl acrylate, stearyl acrylate, isobornyl acrylate, cyclohexyl acrylate), and other functional monomers (2-methoxyethyl acrylate, methoxyethylene glycol acrylate, 2-ethoxyethyl acrylate, tetrahydrofurfuryl acrylate, benzyl acrylate, ethyl carbithol acrylate, phenoxyethyl acrylate, N,N-dimethylaminoethyl acrylate, N,N-dimethylaminopropyl acrylamide, N,N-dimethylacrylamide, acryloyl morpholine, N-isopropyl acrylamide, N,N-diethylacrylamide, N-vinylpyrrolidone, 2-(perfluorooctyl)ethyl acrylate, 3-perfluorohexyl-2-hydroxypropyl acrylate, 3-perfluorooctyl-2-hydroxypropyl acrylate, 2-(perfluorodecyl) ethyl acrylate, 2-(perfluoro-3-methylbutyl)ethyl acrylate), 2,4,6-tribromophenol acrylate, 2,4,6-tribromophenol methacrylate, 2-(2,4,6-tribromophenoxy)ethyl acrylate), and 2-ethylhexyl acrylate.

Examples of the "difunctional monomer" may include tri(propylene glycol) diacrylate, trimethylolpropane-diaryl ether, and urethane acrylate.

Examples of the "polyfunctional monomer" may include trimethylolpropane triacrylate, dipentaerythritol penta- and hexa-acrylates, and ditrimethylolpropane tetraacrylate.

Among these, the preferred resin compositions to compose the transfer material 36 include 2-hydroxyethyl acrylate, acrylamorpholine, glycerol acrylate, polyether acrylate, N-vinylformamide, N-vinylpyrrolidone, N-vinylcaprolactone, ethoxydiethylene glycol acrylate, methoxy triethylene glycol acrylate, polyethylene glycol acrylate, EO-modified trimethylolpropane triacrylate, EO-modified bisphenol A diacrylate, aliphatic urethane oligomer, and polyester oligomer.

Examples of the "initiator" may include 2,2-dimethoxy-1,2-diphenylethan-1-one, 1-hydroxy-cyclohexylphenyl ketone, and 2-hydroxy-2-methyl-1-phenylpropan-1-one.

Examples of the "filler" may include both inorganic and organic fine particles. Inorganic fine particles may include metal oxide fine particles such as SiO₂, TiO₂, ZrO₂, SnO₂, and Al₂O₃.

Examples of the "functional additive" may include leveling agents, surface modifiers, and defoaming agents.

By forming the moth-eye structure composed of fine uneven structures as structures 12, the film laminate 1 achieves a high level of anti-reflection function. Here, the anti-reflection performance of the combination of the front surface and the back surface of the film laminate 1 is 5% or less, preferably 1% or less, and even more preferably 0.5% or less. The light source for surgery has an illuminance of 100,000 lx or more, and even a few percent of reflected light can be blinding, so it is necessary to suppress reflection as much as possible. Furthermore, the film laminate 1 can be well used as a face shield, an eye shield, and protective clothing for medical use, because the film laminate 1 having a layer of an anti-reflection film composed of a moth-eye structure having multiple structures with a pitch smaller than the wavelength of visible light as structures 12 on a transparent base body 11, has a low wavelength dependence, a low angle dependence, and a high anti-reflection performance.

In addition, the film laminate 1 can be made to have anti-fogging properties by using a hydrophilic resin to form the multiple structures having a pitch smaller than the wavelength of visible light which constitute the anti-reflection layer composed of a moth-eye structure.

Furthermore, the film laminate 1 can have even better anti-reflection performance by forming an anti-reflection layer composed of a moth-eye structure formed on both sides of the transparent base body 11.

### MANUFACTURING STEPS OF FILM-SHAPED MEMBERS

Next, the steps for manufacturing the film-shaped members 2 with a moth-eye structure formed thereon will be explained. The moth-eye structure is formed by using a roll master exposure device in which a pattern corresponding to the moth-eye structure is formed to transfer the pattern.

### ROLL MASTER

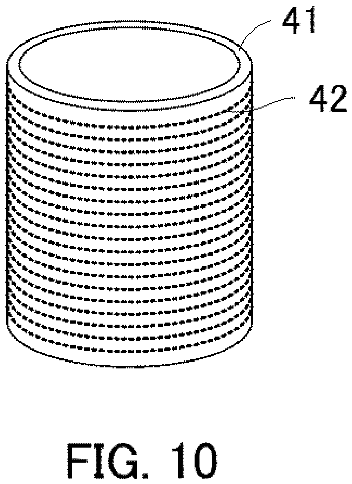

As shown in FIG. 10, a roll master 41 has a round columnar or cylindrical shape, and the round columnar or cylindrical surface is used as the forming surface for forming the plurality of structures 12 on the base body surface. On this forming surface, e.g., predetermined structures 42 are provided in a two-dimensional array by dry etching, wet etching, or the like. The structures 42 have a concave or convex shape with respect to the forming surface. The material of the roll master 41 can be glass, e.g., but is not limited to this material.

The multiple structures 42 arranged on the forming surface of the roll master 41 and the multiple structures 12 arranged on the surface of the base body 11 described above are in an inverted concave-convex relationship. In other words, the shape, arrangement, and pitch of the structures 42 on the roll master 41 are the same as those of the structures 12 on the base body 11.

### TRANSFER STEP 1

As shown in FIG. 11A, after applying the transfer material 36 to one side of the base body 11, the side to which the transfer material 36 is applied is brought into close contact with the roll master 41 in which a pattern corresponding to the moth-eye structure is formed. Next, the transfer material 36 is cured by irradiating it with an energy ray such as an ultraviolet ray from an energy ray source 37, and then the base body 11, which is integrated with the cured transfer material 36, is peeled off. This results in the film-shaped member 2 having multiple structures 12 formed on one side of the base body 11, as shown in FIG. 11B. If necessary, the base layer 13 may be formed between the structures 12 and the base body 11.

The energy ray source 37 may be any source capable of emitting energy rays, such as electron rays, ultraviolet rays, infrared rays, laser rays, visible rays, ionizing radiation rays (such as X-rays, alpha rays, beta rays, gamma rays), microwaves, or radio waves, and is not limited to any particular type.

### TRANSFER STEP 2

When the film-shaped member 2 with multiple structures 12 formed on both sides of a base body 11 is required, as shown in FIG. 11C, after bringing the roll master 41 for the moth-eye structure and the transfer material 36 applied to the surface of the base body 11 opposite to the surface on which the structures are formed into close contact, the transfer material 36 is cured by irradiating it with an energy ray such as an ultraviolet ray from the energy ray source 37. Next, the base body 11, which is integrated with the cured transfer material 36, is peeled off. This results in the film-shaped member 2 having multiple structures 12 formed on both sides of the base body 11, as shown in FIG. 11D. If necessary, the base layer 13 may be formed between the structures 12 and the base body 11.

The resin composition used as the transfer material 36 in this transfer step 2 can be the same as that used in the aforementioned transfer step 1.

In addition, a protective film can be attached to the surface of the film-shaped member 2 obtained in transfer step 1 or transfer step 2. This prevents the structures 12 of the film-shaped member 2 from being damaged in subsequent processes or during transportation.

### SHAPE-FORMING STEP

The film-shaped member 2 obtained as described above is cut into the required shape according to the protective equipment 21 to which the film-shaped member 2 is to be attached, the insertion holes 4 described above are formed, and the slits 10 and the tabs 20 are processed as appropriate. The insertion holes 4 and the tabs 20 can be processed using a numerically controlled cutting machine, a laser processing device, or a punching press. The use of a punching press is suitable because it allows the formation of the insertion holes 4 and the cutting to the specified shape to be performed in a single step.

### LAMINATING STEP

Next, the film-shaped member 2 is laminated by inserting the protruding members 5 into the insertion holes 4 of the film-shaped member 2. There is no particular limit to the number of layers of the film-shaped member 2, and this can be set according to the intended use of the protective equipment. However, the more layers there are, the greater the impact on optical properties, so for applications where a clear field of vision is required, it is better to have fewer layers. In medical applications, there are cases where the entire film laminate 1 and protective equipment 21 must be disposable for hygienic reasons, so two or three layers are preferable. As mentioned above, the laminating method involves inserting the protruding members 5 through the insertion holes 4, starting with the bottommost film-shaped member 2. Each film-shaped member 2 is laminated at a predetermined interval using the engagement portions, and the entire front and back surfaces are exposed. This results in the film laminate 1.

### STERILIZATION STEP

Next, the film laminate 1 is sterilized. Sterilization can be carried out using gas sterilization with, e.g., ethylene oxide gas or radiation sterilization with, e.g., gamma rays. The film laminate 1 has a predetermined gap between each film-shaped member 2, so that the entire front and back surfaces are exposed. Therefore, it is possible to expose all the surfaces of all the film-shaped members 2 to the sterilizing gas. Furthermore, in the film laminate 1, even if the film-shaped members 2 are formed using a material that does not allow the penetration of sterilizing gas such as ethylene oxide gas, it is still possible to sterilize all the surfaces sufficiently.

Sterilization is carried out after the film laminate 1 is formed, but before it is attached to the protective equipment 21 described below; however, sterilization may also be carried out after the film laminate 1 is attached to the protective equipment 21, or at both of these times.

During the use of the film laminate 1, when the topmost film-shaped member 2 becomes contaminated, by peeling off the contaminated topmost film-shaped member 2, the entire surface of the next film-shaped member 2 is exposed. The newly exposed film-shaped member 2 has been fully sterilized, so the risk of infection to the patient, the examinee, and the medical personnel can be reduced.

Also, the film-shaped member 2 can be peeled off simply by removing the insertion holes 4 from the protruding member 5, so there is no risk of damaging the moth-eye structure formed on the film-shaped member 2. In this respect, in a laminated structure where the film-shaped members 2 are bonded together with a pressure-sensitive adhesive, when the film-shaped member 2 is peeled off, there is a risk of mechanical damage such as damage to the interface between the pressure-sensitive adhesive layer and the film-shaped members 2 or cohesion failure between the pressure-sensitive adhesive layers, which may damage the moth-eye structure and have a negative impact on visibility, depending on the bonding strength. In addition, the scattering of adhesive residue and the generation of outgases from the pressure-sensitive adhesive layer are particularly undesirable in medical settings.

In a laminated structure in which the film-shaped members 2 are bonded to each other by welding, there is also a risk of damage to the moth-eye structure, which affects visibility, because the bonding between the film-shaped members 2 involves physical destruction when they are peeled off. In addition, the scattering of residue due to the destruction of the welding points is particularly undesirable in medical settings.

Since the film laminate 1 does not use pressure-sensitive adhesive and the film-shaped members 2 are not welded, there are no risks associated with them, so the film laminate 1 can be used safely.

### PROTECTIVE EQUIPMENT

The film laminate 1 is attached to the protective equipment 21. There is no particular restriction on the protective equipment 21, as long as it is used to ensure visibility by peeling off the film-shaped member 2; examples of the protective equipment 21 may include medical protective clothing (coveralls), medical face shields, medical eye shields, medical displays, helmet visors, protective glasses for painting, and chemical protective clothing used in times of disaster, among others.

The protective equipment 21 provided with the film laminate 1 has the film laminate 1 attached to the area corresponding to the face or eyes of the user wearing the protective equipment 21. If the surface of the film laminate 1 becomes contaminated during the use of the protective equipment 21, the topmost film-shaped member 2 can be peeled off to quickly restore visibility while avoiding contact with the contaminant. As the front surface and the back surface of each layer of the film-shaped member 2 in the film laminate 1 are sterilized, the sterilized surface always appears on the front surface, not only at the beginning of use but also after peeling. This reduces the risk of infection to the patient, the examinee, and other medical personnel in medical applications. In addition, since the entire back surface of the peeled-off film-shaped member 2 has also been sterilized, there is no risk of exposing the patient, examinee, and medical personnel to unsterilized areas when the film-shaped member 2 is peeled off or disposed of.

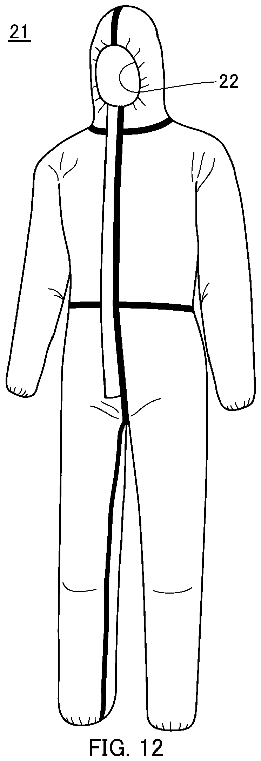

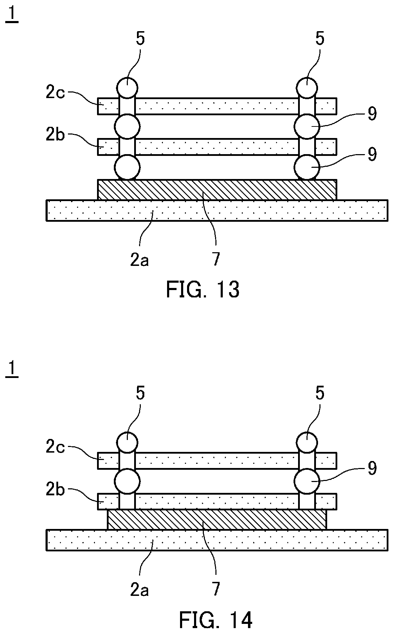

There is no particular restriction on the attaching method of the film laminate 1, and the method may be decided as appropriate according to the specifications of the protective equipment 21 to be used. FIG. 12 is an exterior perspective view illustrating a protective suit for medical use as an example of protective equipment 21. For example, as shown in FIG. 12, the protective equipment 21 has an opening 22 in a position corresponding to the face of the user, and the film laminate 1 is attached to this opening 22. In the film laminate 1 shown in FIG. 13, three film-shaped members 2 are laminated; specifically, the base 7 is connected to the bottommost film-shaped member 2a (i.e., the film-shaped member 2a that is positioned closest to the user's face) by means such as adhesion, and the two protruding members 5 provided on the base 7 are inserted through the intermediate film-shaped member 2b and the topmost film-shaped member 2c. The number of layers of the film-shaped members 2 that constitute the film laminate 1 according to the present technology is not limited to three. The number of the protruding members 5 is also not limited to two.

The intermediate film-shaped member 2b is separated from the base 7 by the protrusions 9, and the entire front and back surfaces are exposed, and the entire front surface of the base 7 is exposed, thereby enabling the sterilization process. In addition, as shown in FIG. 14, the intermediate film-shaped member 2b may be in contact with the base 7. Even in the configuration shown in FIG. 14, in which the protruding members 5 are inserted into the film-shaped member 2b without bonding the film-shaped member 2b to the base 7, if the back surface of the film-shaped member 2b is in gentle contact with the base 7, it is possible to allow sterilizing gas to enter therebetween.

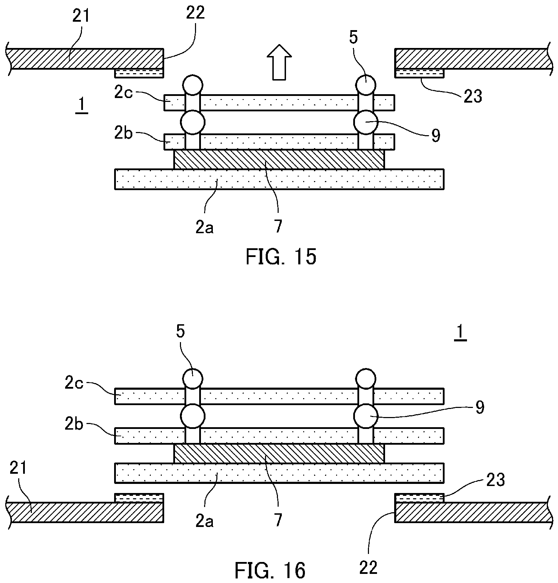

As shown in FIG. 15, in the film laminate 1, the bottommost film-shaped member 2a on which the base 7 is provided is formed to be larger than the opening 22. In addition, an adhesive layer 23 using double-sided tape is provided on the outer edge of the back side of the opening 22. The topmost film-shaped member 2c and the intermediate film-shaped member 2 of the film laminate 1 are inserted through the opening 22 from the back side of the protective equipment 21, and the bottommost film-shaped member 2a is adhered to the periphery of the opening 22 via the adhesive layer 23. This allows the film laminate 1 to be attached to the protective equipment 21.

Alternatively, as shown in FIG. 16, the bottommost film-shaped member 2a may be attached to the front surface side of the protective equipment 21. In this case, the adhesive layer 23 using double-sided tape or the like is provided on the outer edge of the front surface side of the opening 22. The bottommost film-shaped member 2a of the film laminate 1 is then attached to the front surface side of the opening 22 via the adhesive layer 23. In the configuration shown in FIG. 16, the intermediate film-shaped member 2b and the topmost film-shaped member 2c having an area larger than the opening 22 can be used.

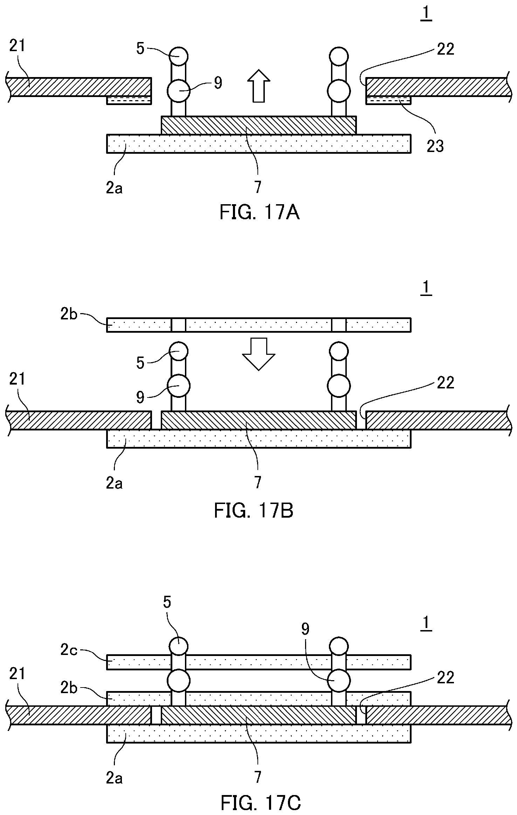

In addition, as shown in FIG. 17, the film laminate 1 and the protective equipment 21 provided with the film laminate 1 may be simultaneously completed by connecting the bottommost film-shaped member 2a having the base 7 connected thereon to the opening 22 of the protective equipment 21 (FIG. 17A), laminating the intermediate film-shaped member 2b (FIG. 17B), and then laminating the topmost film-shaped member 2c (FIG. 17C).

Also, although not shown in the figure, the film laminate 1 and the protective equipment 21 provided with the film laminate 1 may be simultaneously completed by attaching the base 7 to the vicinity of the opening 22 of the protective equipment 21 by means such as adhesion, and sequentially laminating the film-shaped members 2 through the protruding members 5 erected on the base 7.

### EXAMPLES

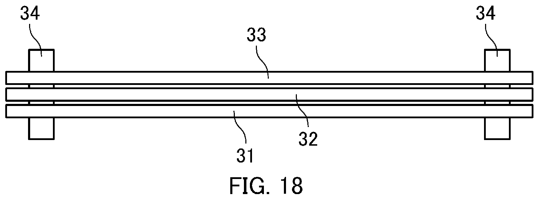

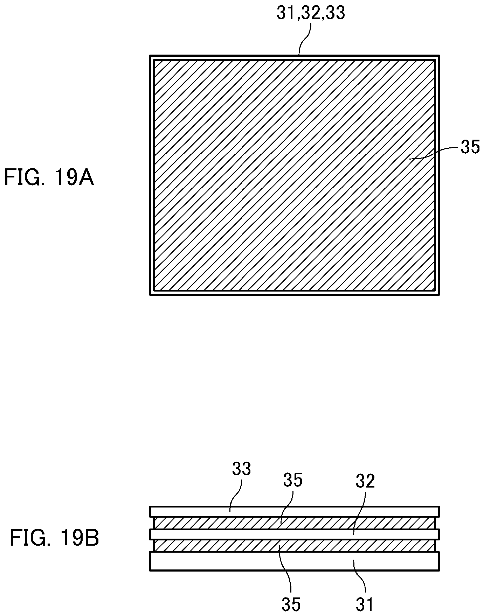

Next, examples of the present technology will be explained. In the following, samples of the example of the film laminate according to the present technology were prepared by laminating the film-shaped members 2 using the protruding members 5 with engagement portions (hereinafter referred to as "hook members 34") as shown in FIG. 18, and samples of the comparative example were prepared by laminating the film-shaped members 2 bonded with pressure-sensitive adhesive 35 as shown in FIG. 19.

In these examples, film laminate samples with different film-shaped member laminating means and gap widths were prepared and evaluated for gas sterilization, visibility (transmittance %, haze %), ease of peeling, and risk of peeling off the moth-eye structure when peeling off the film (hereafter referred to as "contamination evaluation").

For the evaluation of gas sterilization, evaluation samples were prepared in the form of 100 x 100 mm films, and stickers (sterilization labels EO-L manufactured by Nippon Oil & Energy Technology Center Co., Ltd.) that reveal the word "sterilized" when they react with EOG were pasted to five locations: the four corners and the center of the area defined by the laminating means (the hook member 34 or the pressure-sensitive adhesive 35).

The sterilization conditions were as follows.
Temperature: 50°C
Humidity: 50% RH
Exposure time: 8 hours
Chamber pressure: 100 kPa, decompression pressure: -85 kPa
Flushing: 5 times

The results were evaluated as follows: if the word "sterilized" appeared, the result was marked as G (good: sterilization was effective); if the word "sterilized" did not appear, the result was marked as B (bad: sterilization was not effective).

For the visibility judgment (transmittance [%] and haze [%]), the transmittance [%] and haze [%] were determined using a haze meter (HM-150N) manufactured by MURAKAMI COLOR RESEARCH LABORATORY using the double beam method (JIS K 7361, JIS K 7136). A ϕ150mm integrating sphere was used. The visibility judgment criteria were G (good) for haze of less than 1.5%, and B (bad) for haze of 1.5% or more.

The ease of peeling evaluation is a sensory evaluation of the ease of peeling in the TD direction and was evaluated in order of ease of peeling as G (good), I (intermediate), and B (bad).

The contamination evaluation is an evaluation of the risk of the moth-eye structure peeling off when the film is peeled off: a mark of G is given to a film with no risk of the moth-eye structure peeling off; a mark of B is given to a film where the peeling off of the moth-eye structure was clearly predictable; and a mark of I is given to a film where the risk of the moth-eye structure peeling off was not clear but could be predicted to some extent.

### Example 1

In Example 1, a first film 31 having a moth-eye structure formed on a PET base material with a thickness of 188 µm, a second film 32 having a moth-eye structure formed on a PET base material with a thickness of 100 µm, and a third film 33 having a moth-eye structure formed on a PET base material with a thickness of 100 µm were laminated together using the hook members 34 to prepare a film laminate sample. In Example 1, the films were laminated so that no gaps were created between the first film 31 and the second film 32, and between the second film 32 and the third film 33, using the hook member 34 (gap width of approximately 0 mm).

### Example 2

The film laminate sample for Example 2 is the same as Example 1, except that the films are laminated so that the gap width between the first film 31 and the second film 32 and the gap width between the second film 32 and the third film 33 are 0.038 mm.

### Example 3

The film laminate sample for Example 3 is the same as Example 1, except that the films are laminated so that the gap width between the first film 31 and the second film 32 and the gap width between the second film 32 and the third film 33 are 0.050 mm.

### Example 4

The film laminate sample for Example 4 is the same as Example 1, except that the films are laminated so that the gap width between the first film 31 and the second film 32 and the gap width between the second film 32 and the third film 33 are 0.075 mm.

### Example 5

The film laminate sample for Example 5 is the same as Example 1, except that the films are laminated so that the gap width between the first film 31 and the second film 32 and the gap width between the second film 32 and the third film 33 are 0.100 mm.

### Example 6

The film laminate sample for Example 6 is the same as Example 1, except that the films are laminated so that the gap width between the first film 31 and the second film 32 and the gap width between the second film 32 and the third film 33 are 0.150 mm.

### Example 7

The film laminate sample for Example 7 is the same as Example 1, except that the films are laminated so that the gap width between the first film 31 and the second film 32 and the gap width between the second film 32 and the third film 33 is 0.300 mm.

### Example 8

The film laminate sample for Example 8 is the same as Example 1, except that the films are laminated so that the gap width between the first film 31 and the second film 32 and the gap width between the second film 32 and the third film 33 are 0.500 mm.

### Example 9

The film laminate sample for Example 9 is the same as Example 1, except that the films are laminated so that the gap width between the first film 31 and the second film 32 and the gap width between the second film 32 and the third film 33 are 1.0 mm.

### Example 10

The film laminate sample for Example 10 is the same as Example 1, except that the films are laminated so that the gap width between the first film 31 and the second film 32 and the gap width between the second film 32 and the third film 33 are 2.0 mm.

### Example 11

The film laminate sample for Example 11 is the same as Example 1, except that the films are laminated so that the gap width between the first film 31 and the second film 32 and the gap width between the second film 32 and the third film 33 are 3.0 mm.

### Comparative Example 1

In Comparative Example 1, a first film 31 made of a PET base material with a thickness of 188 µm, a second film 32 made of a PET base material with a thickness of 50 µm, and a third film 33 made of a PET base material with a thickness of 50 µm were laminated together by using an acrylic pressure-sensitive adhesive 35 to paste the full surfaces of all three films together to prepare a film laminate sample. As shown in FIG. 19, the film laminate sample for Comparative Example 1 has a gap width of 0 mm due to the pressure-sensitive adhesive layer being present between the first film 31 and the second film 32 and between the second film 32 and the third film 33.

### Comparative Example 2

In Comparative Example 2, a first film 31 having a moth-eye structure formed on a PET base material with a thickness of 188 µm, a second film 32 having a moth-eye structure formed on a PET base material with a thickness of 100 µm, and a third film 33 having a moth-eye structure formed on a PET base material with a thickness of 100 µm were laminated together by using an acrylic pressure-sensitive adhesive 35 to paste the full surfaces of all three films to prepare a film laminate sample. The film laminate sample for Comparative Example 2 has a gap width of 0 mm due to the pressure-sensitive adhesive layer being present between the first film 31 and the second film 32 and between the second film 32 and the third film 33.

### Comparative Example 3

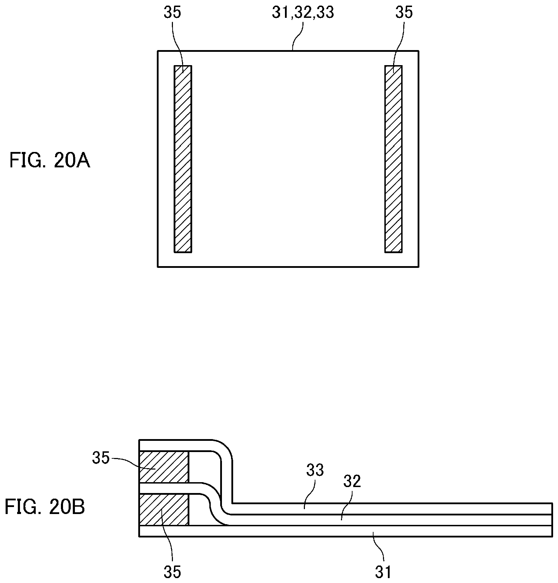

In Comparative Example 3, a first film 31 having a moth-eye structure formed on a PET base material with a thickness of 188 µm, a second film 32 having a moth-eye structure formed on a PET base material with a thickness of 100 µm, and a third film 33 having a moth-eye structure formed on a PET base material with a thickness of 100 µm were laminated together using an acrylic pressure-sensitive adhesive 35 applied to one side edge of each of the first film 31 to third film 33 to prepare a film laminate sample. As shown in FIG. 20, in the film laminate sample for Comparative Example 3, adjacent films were pasted together by bending one of the films so that the gap width between the first film 31 and the second film 32 and the gap width between the second film 32 and the third film 33 were approximately 0 mm.

**Table 1**

| | EX. 1 | EX. 2 | EX. 3 | EX. 4 | EX. 5 | EX. 6 | EX. 7 |
|---|---|---|---|---|---|---|---|
| first film | PET188+moth-eye | PET188+moth-eye | PET188+moth-eye | PET188+moth-eye | PET188+moth-eye | PET188+moth-eye | PET188+moth-eye |
| second film | PET100+moth-eye | PET100+moth-eye | PET100+moth-eye | PET100+moth-eye | PET100+moth-eye | PET100+moth-eye | PET100+moth-eye |
| third film | PET100+moth-eye | PET100+moth-eye | PET100+moth-eye | PET100+moth-eye | PET100+moth-eye | PET100+moth-eye | PET100+moth-eye |
| gap width | 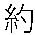 0mm | 0.038mm | 0.050mm | 0.075mm | 0.100mm | 0.150mm | 0.300mm |
| gas sterilization | G | G | G | G | G | G | G |
| transmittance | 95.9% | 95.8% | 95.8% | 95.8% | 95.8% | 95.8% | 95.8% |
| haze | 0.6% | 0.7% | 0.7% | 0.7% | 0.7% | 0.7% | 0.7% |
| visibility | G | G | G | G | G | G | G |
| ease of peeling | G | G | G | G | G | G | G |
| laminating means | hook member | hook member | hook member | hook member | hook member | hook member | hook member |
| material | plastic | plastic | plastic | plastic | plastic | plastic | plastic |
| contamination | G | G | G | G | G | G | G |
| total evaluation | G | G | G | G | G | G | G |

| | EX. 8 | EX. 9 | EX. 10 | EX. 11 | COMP. 1 | COMP. 2 | COMP. 3 |
|---|---|---|---|---|---|---|---|
| first film | PET188+moth-eye | PET188+moth-eye | PET188+moth-eye | PET188+moth-eye | PET188 | PET188+moth-eye | PET188+moth-eye |
| second film | PET100+moth-eye | PET100+moth-eye | PET100+moth-eye | PET100+moth-eye | PET50 | PET100+moth-eye | PET100+moth-eye |
| third film | PET100+moth-eye | PET100+moth-eye | PET100+moth-eye | PET100+moth-eye | PET50 | PET100+moth-eye | PET100+moth-eye |
| gap width | 0.500mm | 1.0mm | 2.0mm | 3.0mm | 0mm | 0mm | 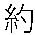 0mm |
| gas sterilization | G | G | G | G | B | B | G |
| transmittance | 95.8% | 95.8% | 95.8% | 95.7% | 87.0% | 95.3% | 95.8% |
| haze | 0.6% | 0.6% | 0.6% | 0.6% | 1.8% | 2.2% | 0.6% |
| visibility | G | G | G | G | B | B | G |
| ease of peeling | G | G | G | G | G | G | G |
| laminating means | hook member | hook member | hook member | hook member | adheasive (full pasting) | adheasive (full pasting) | adheasive (partial pasting) |
| material | plastic | plastic | plastic | plastic | acrylic adhesive | acrylic adhesive | acrylic adhesive |
| contamination | G | G | G | G | - | B | I |
| total evaluation | G | G | G | G | B | B | I |

As shown in Table 1, in the film laminate samples for Examples 1 to 11, the effect of sterilization was confirmed since the word "sterilized" appeared on the sticker. In Example 1, even if there is no apparent gap between the first film 31 and the second film 32 or between the second film 32 and the third film 33 when viewed from the side, these films are not in close contact with each other but are only slightly touching, and the sterilizing gas can pass through the layers. In other words, this example shows that the sterilization effect is achieved by laminating the film-shaped members with a certain gap (about 0 to 3 mm) between them.

In addition, in film laminate samples for Examples 1 to 11, the first film 31, the second film 32, and the third film 33 can be peeled apart by releasing the engagement with the hook member 34, and the contamination evaluation is G, i.e., there is no risk of peeling occurring due to the force acting in the direction of peeling off the moth-eye structure formed on the film.

In Comparative Example 3 in which the pressure-sensitive adhesive 35 is provided on one side edge of the first film 31 to the third film 33 and the films are laminated, the same sterilization effect as in Examples 1 to 11 was confirmed, but the contamination evaluation was I (intermediate) because the first film 31 to the third film 33 are laminated together using the pressure-sensitive adhesive so that there is a risk of peeling off the moth-eye structure because a force acts in the direction of peeling off the moth-eye structure formed on the film when the first film 31, the second film 32 and the third film 33 are peeled off. In addition, in Comparative Example 2, since the pressure-sensitive adhesive 35 is applied to the full surfaces of the first film 31 to the third film 33 to laminate the films, the contamination evaluation was B (bad), in other words, the peeling off of the moth-eye structure is clearly predictable. Comparative Example 1 is not subject to the contamination evaluation since the moth-eye structure is not formed on the first film 31 to the third film 33.

### REFERENCE SIGNS LIST

1: film laminate, 2: film-shaped member, 3: protruding members, 4: insertion hole, 7: base, 9: protrusions, 10: slit, 11: base body, 12: structure, 13: base layer, 20: tab, 21: protective equipment, 22: opening, 23: adhesive layer, 36: transfer material, 37: energy ray source, 41: roll master, 42: structure

## Claims

1. A film-laminate, comprising:
a plurality of film-shaped members laminated in a separable manner; and
columnar protruding members protruding in the direction of lamination of the film-shaped members and holding the film-shaped members at a predetermined interval, wherein
the protruding members have engagement portions on the outer circumference of the protruding members to which the film-shaped members are engaged at the predetermined interval,
the film-shaped members have insertion holes through which the protruding members are inserted and removed as desired, and
the film-shaped members are held at the predetermined interval and laminated by the protruding members being inserted through the insertion holes and the film-shaped members being engaged with the engagement portions.

2. The film laminate according to claim 1, wherein the engagement portions are protrusions that extend in the circumferential direction of the protruding members, and the film-shaped members are engaged between the protrusions.

3. The film laminate according to claim 2, wherein
a plurality of the protrusions are provided, and the diameter of the protrusions successively increases from the protrusions on the tip side of the protruding members to the protrusions on the root side of the protruding members, and
the insertion holes have an aperture diameter corresponding to the diameter of the protrusion at the position where the film-shaped member is engaged.

4. The film laminate according to claim 2 or 3, wherein the protrusions are cylindrical, ring-shaped, or spherical.

5. The film laminate according to claim 1, wherein the engagement portion is a groove formed around the protruding members, and the film-shaped members are engaged with the groove.

6. The film laminate according to any one of claims 2, 3, and 5, wherein the protrusions are formed as spherical protrusions formed at least at the tips of the protruding members.

7. The film laminate according to any one of claims 1 to 3, and 5, wherein the film-shaped members are provided with one or more slits around the insertion holes.

8. The film laminate according to any one of claims 1 to 3, and 5, wherein the insertion holes are formed on the outer edge portion of the film-shaped member.

9. The film laminate according to any one of claims 1 to 3, and 5, wherein the film-shaped members are provided with a plurality of structures on at least one side of a flexible transparent base material with a pitch smaller than the wavelength of visible light.

10. A method for manufacturing a film laminate, comprising:
a step of forming film-shaped members having insertion holes;
a step of forming protruding members having engagement portions that engage with the film-shaped members; and
a step of inserting the protruding members through the insertion holes of the film-shaped members, causing the film-shaped members to be engaged with the engagement portions, and laminating the film-shaped members at a predetermined interval.

11. The method for manufacturing a film laminate according to claim 10, further comprising a step of sterilizing the film-shaped members being held at the predetermined interval.

12. Protective equipment in which a film laminate is attached to a part corresponding to the face or eyes of a user, wherein
the film laminate is the film laminate according to any one of claims 1 to 3, and 5.

13. The protective equipment according to claim 12, wherein the protective equipment is protective clothing, a face shield, an eye shield, or a helmet.

14. A method for manufacturing protective equipment in which a film laminate is attached to a part corresponding to the face or eyes of a user, comprising:
a step of forming a film laminate; and
a step of attaching the film laminate to protective equipment, wherein
the film laminate is the film laminate according to any one of claims 1 to 3, and 5.
